# EUROPÄISCHE PATENTANMELDUNG

(11) **EP 0 600 367 A1**
(43) Veröffentlichungstag der Anmeldung: **08.06.1994**
(21) Anmeldenummer: 93118975.7
(22) Anmeldetag: 25.11.1993
(51) Int. Cl.: C07B 61/00, C07C 1/34, C07C 17/26, C07C 15/46, C07C 25/28, C07C 41/30, C07C 43/215, C07C 51/353, C07C 63/64, C07C 67/293, C07C 69/157

(54) **Verfahren zur Herstellung von aromatischen Vinylverbindungen**

(30) Priorität: 01.12.1992 DE 4240321
(71) Anmelder: BASF Aktiengesellschaft, D-67063 Ludwigshafen (DE)
(72) Erfinder: Ditrich, Klaus, Dr., D-67098 Bad Duerkheim (DE)

(57) **Zusammenfassung**

Herstellung von aromatischen Vinylverbindungen der allgemeinen Formel I

Ar―CH=CHR I

in der Ar einen aromatischen Rest bedeutet und R für Wasserstoff, C₁-C₄-Alkyl oder einen Phenyl- oder substituierten Phenylrest steht, indem man einen Alkohol der Formel II

Ar―CH₂OH II

oder einen Ester der Formel III 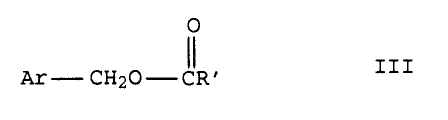
in der R' für Wasserstoff oder C₁-C₄-Alkyl steht,
in wäßriger Lösung mit einem Triarylphosphin und einer starken Säure und danach in Gegenwart einer Mineralbase mit einem Aldehyd der Formel IV

R―CHO IV

umsetzt.

## Beschreibung

Die vorliegende Erfindung betrifft ein neues Verfahren zur Herstellung von aromatischen Vinylverbindungen der allgemeinen Formel I

Ar―CH=CHR I

in der Ar einen aromatischen Rest bedeutet und R für Wasserstoff, C₁-C₄-Alkyl oder einen Phenyl- oder substituierten Phenylrest steht.

Aus Arch. Pharm. 322 (1989) 159 ist die Umsetzung eines 1-Tetralols mit Triphenylphosphoniumbromid in Benzol zum entsprechenden Tetraryltriphenylphosphoniumbromid bekannt. Triphenylphosphoniumbromid wird dazu in einer gesonderten Reaktion hergestellt.

In Synth. Commun. 6 (1976) 53 wird die Herstellung einiger p-sub-stituierter Styrolderivate beschrieben. Aus parasubstituierten Benzylhalogeniden werden durch Umsetzung mit Triphenylphosphin in Chloroform Benzylphosphoniumsalze hergestellt und isoliert. Diese werden anschließend mit wäßriger Formalinlösung und Natriumhydroxid zu den entsprechenden Styrolderivaten umgesetzt.

Der Erfindung lag die Aufgabe zugrunde, ein Verfahren zur Herstellung von aromatischen Vinylverbindungen bereitzustellen, das die Isolierung von Zwischenverbindungen vermeidet.

Demgemäß wurde eine Verfahren zur Herstellung von aromatischen Vinylverbindungen der allgemeinen Formel I

Ar―CH=CHR I

in der Ar einen aromatischen Rest bedeutet und R für Wasserstoff, C₁-C₄-Alkyl oder einen Phenyl- oder substituierten Phenylrest steht, gefunden, das dadurch gekennzeichnet ist, daß man einen Alkohol der Formel II

Ar―CH₂OH II

oder einen Ester der Formel III 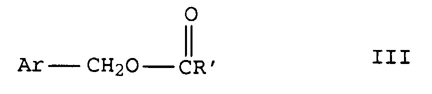
in der R' für Wasserstoff oder C₁-C₄-Alkyl steht,
in wäßriger Lösung mit einem Triarylphosphin und einer starken Säure und danach in Gegenwart einer Mineralbase mit einem Aldehyd der Formel IV

R―CHO IV

umsetzt.

Die folgende Reaktionsgleichung gibt die Reaktion schematisch für die Umsetzung eines Benzylalkohols wieder: 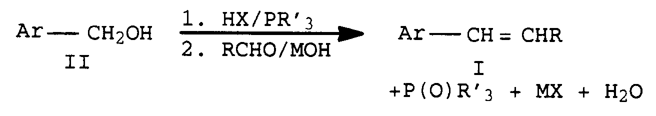
- X =: Säureanion
- R' =: Aryl
- M =: Alkalimetall
Die Alkohole II und die Ester III sind bekannt oder nach bekannten Methoden herstellbar. Sie umfassen u.a. Benzylalkohole oder deren Ester, die einen oder mehrere Substituenten am aromatischen Ring tragen können. Als Substituenten kommen vorzugsweise C₁-C₄-Alkoxy wie Methoxy, Ethoxy, iso-Propoxy und n-Propoxy, C₁-C₄-Alkylthio wie Methylthio, Ethylthio und n-Propylthio, C₁-C₆-Alkyl wie Methyl, Ethyl, iso-Propyl und n-Propyl, C₂-C₆-Alkenyl wie Vinyl, C₂-C₆-Alkinyl, Carboxyl, Nitro, Cyano, Hydroxy, Halogen wie Fluor, Chlor, Brom und Jod sowie halogensubstituiertes C₁-C₄-Alkyl wie Trifluormethyl in Betracht. Sind weitere benzylische Hydroxyl- oder Estergruppen am Aromaten vorhanden, kann die Reaktion auch mehrfach an diesem Aromaten ablaufen. Weiterhin sind Naphthylmethylalkohole sowie Anthracenylmethylalkohole zu nennen, die ebenfalls substituiert sein können.

Die Alkohole II bzw. die Ester III werden mit Triarylphosphinen umgesetzt. Es kommen aromatische Phosphine mit Substituenten am Arylrest in Betracht, bevorzugt wird aber Triphenylphosphin.

Der Alkohol II bzw. der Ester III und das Phosphin werden mit starken Säuren umgesetzt. Die Säuren haben üblicherweise einen pKₐ-Wert von unter null. Es handelt sich dabei z.B. um Mineralsäuren wie Chlorwasserstoffsäure, Bromwasserstoffsäure und Schwefelsäure, um Carbonsäuren wie Essigsäure und Trifluoressigsäure und um Sulfonsäuren wie p-Toluolsulfonsäure. Von der Verwendung von Salpetersäure ist abzuraten, da diese zu unkontrollierten explosionsartigen Reaktionen führen kann.

In der Regel wird das Phosphin im geringen stöchiometrischen Überschuß von 1:1 bis 3:1 in Bezug auf den Alkohol II bzw. den Ester III verwendet. Die Säure wird im allgemeinen in größerem Überschuß eingesetzt, d.h. etwa 1,5 bis 20 Äquivalente Säure pro Äquivalent Alkohol.

Zur Umsetzung können der Ausgangsstoff, das Phosphin und die Säure in wäßriger Lösung gemischt werden und zur Beschleunigung der Umsetzung für 1 bis 3 Stunden auf 50 bis 100°C erhitzt werden.

Zur weiteren Umsetzung wird dann die so erhaltene Reaktionslösung mit einem Aldehyd IV O=CH-R versetzt, wobei R für Wasserstoff, C₁-C₄-Alkyl oder einen Phenyl- oder substituierten Phenylrest wie Alkylphenyl steht. Vorzugsweise handelt es sich dabei um Formaldehyd, es kommen aber auch z.B. Acetaldehyd, n-Propionaldehyd und Benzaldehyd in Betracht.

Der Aldehyd wird vorzugsweise in einer Mischung mit Wasser eingesetzt, die in der Regel 20- bis 40 Gew.-% Aldehyd enthält. Das molare Verhältnis von Aldehyd IV zu Alkohol II bzw. Ester III liegt meistens bei 1:1 bis 50:1, bevorzugt bei 3:1 bis 15:1.

Weiterhin wird eine Mineralbase zugesetzt. Erdalkalihydroxide wie Calciumhydroxid und Bariumhydroxid kommen ebenso wie Alkalimetallhydroxide wie Lithiumhydroxid oder vorzugsweise Natriumhydroxid und Kaliumhydroxid in Betracht. Die Basen werden in der Regel als 20 bis 60 %ige wäßrige Lösung eingesetzt.

Die Reaktion kann bei 0 bis 100°C vorgenommen werden, vorzugsweise bei 20 - 70°C. Sie ist im allgemeinen nach 15 Stunden vollständig, oft genügen auch schon 3 Stunden. Die Reaktion kann unter erhöhtem Druck bis zu etwa 10 bar durchgeführt werden, im Normalfall führt man sie aber bei Normaldruck aus.

Die Isolierung der Wertprodukte erfolgt nach bekannten Methoden, etwa durch Abfiltrieren des gebildeten Triarylphosphinoxids, Extraktion mit einem organischen Lösungsmittel und Kristallisation oder Destillation.

Es kann für bestimmte Verfahrensprodukte auch vorteilhaft sein, die Reaktion in Gegenwart eines organischen Lösungsmittels wie Dichlormethan vorzunehmen, wobei die Produkte direkt in die organische Phase extrahiert werden. In dieser Ausführungsvariante können auch Phasentransferkatalysatoren wie quaternäre Ammonium-verbindungen, z.B. Triethylbenzylammoniumchlorid, mitverwendet werden.

Das erfindungsgemäße Verfahren erlaubt die Herstellung von aromatischen Vinylverbindungen aus Alkoholen II bzw. Estern III in einer einfach zu realisierenden Eintopfreaktion in wäßriger Lösung. Die Isolierung von Phosphoniumsalzen entfällt. Weiterhin ist das in der Reaktion gebildete Triarylphosphinoxid leicht abtrennbar.

Die Verfahrensprodukte dienen zur Herstellung von Polymeren. Zum Beispiel kann p-Hydroxystyrol hergestellt werden, das als Polymer als Bindemittel für Photolacke Verwendung findet (ACS Symp.Ser. 412 (1989)).

### Beispiele

### Allgemeine Vorschrift für die Beispiele 1 - 8

Eine Mischung aus 1 mol Alkohol II und 1,05 mol Triphenylphosphin wurde mit 1,5 mol 37 %iger wäßriger Säure versetzt. Die Mischung wurde 2 Stunden auf 85°C erhitzt, mit 500 ml (6,6 mol) 37 %iger wäßriger Formaldehydlösung und 425 ml (8,0 mol) 50 %iger Natronlauge versetzt. Man hielt die Reaktionslösung 3 Stunden bei 60°C. Nach Filtration wurde das Produkt durch Extraktion mit n-Hexan isoliert und destilliert.

| Beispiel | Substituenten am Benzylalkohol | Säure | Ausbeute [%] |
|---|---|---|---|
| 1 | 4-OCH₃ | HBr | 95 |
| 2 | 2-J | HBr | 70 |
| 3 | 3-Cl, 4-Cl | HBr | 94 |
| 4 | 4-Cl | HCl | 100 |
| 5 | 3-OMe, 4-OMe | HBr | 40 |
| 6 | 3-OMe, 4-OMe, 5-OMe | HBr | 75 |
| 7 | 2-OMe, 3-OMe | HBr | 86 |
| 8 | 4-COOH | HCl | 68 |

### Beispiele 9 - 14

In Analogie zu den Beispielen 1 - 8 wurden 1 mol Benzylester III umgesetzt.

In Beispiel 14 wurden die doppelten Mengen der Reagentien für 1 mol Benzylester eingesetzt, da eine zweifache Reaktion unter Bildung von 1,4-Divinylbenzol abläuft.

| Beispiel | Substituenten am aromatischen Rest Ar | R' | Säure | Ausbeute [%] |
|---|---|---|---|---|
| 9 | 4-CH₃ | H | HCl | 99 |
| 10 | 4-CH₃ | CH₃ | HCl | 96 |
| 11 | 2-CH₃ | CH₃ | HCl | 93 |
| 12 | 3-CH₃ | CH₃ | HCl | 87 |
| 13 | 4-F | CH₃ | HBr | 29 |
| 14 | 4-(CH₂OCOCH₃) | CH₃ | HCl | 74 |

### Beispiele 15 - 17

Eine Mischung aus 1 mol Alkohol II und 1,05 mol Triphenylphosphin wurde mit 1,5 mol 37 %iger wäßriger Säure versetzt. Die Mischung wurde 2 h auf 85°C erhitzt mit 5,0 mol Aldehyd IV in 1 l Dichlormethan versetzt. Nach Zugabe von 6 mol Natronlauge in Form einer 50 %igen wäßrigen Lösung wurde das Reaktionsgemisch 14 h bei Raumtemperatur gerührt, mit Dichlormethan extrahiert und chromatographisch aufgearbeitet.

| Beispiel | Substituenten am Benzylalkohol | R | Säure | Ausbeute [%] |
|---|---|---|---|---|
| 15 | 4-OCH₃ | Phenyl | HCl | 50 |
| 16 | 4-OCH₃ | CH₃ | HCl | 52 |
| 17 | 4-OCH₃ | 1-Methylethyl | HCl | 35 |

## Patentansprüche

1. Verfahren zur Herstellung von aromatischen Vinylverbindungen der allgemeinen Formel I
Ar―CH=CHR I
in der Ar einen aromatischen Rest bedeutet und R für Wasserstoff, C₁-C₄-Alkyl oder einen Phenyl- oder substituierten Phenylrest steht, dadurch gekennzeichnet, daß man einen Alkohol der Formel II
Ar―CH₂OH II
oder einen Ester der Formel III 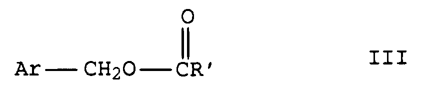 in der R' für Wasserstoff oder C₁―C₄-Alkyl steht,
in wäßriger Lösung mit einem Triarylphosphin und einer starken Säure und danach in Gegenwart einer Mineralbase mit einem Aldehyd der Formel IV
R―CHO IV
umsetzt.

2. Verfahren nach Anspruch 1, dadurch gekennzeichnet, daß man als Phosphin Triphenylphosphin verwendet.

3. Verfahren nach Anspruch 1 oder 2, dadurch gekennzeichnet, daß man als aromatische Verbindung p-Methoxybenzylalkohol und als Aldehyd Formaldehyd verwendet.
